# EUROPÄISCHE PATENTSCHRIFT

(11) **EP 2 014 321 B1**
(45) Veröffentlichungstag und Bekanntmachung des Hinweises auf die Patenterteilung: **29.09.2010**
(21) Anmeldenummer: 07112391.3
(22) Anmeldetag: 12.07.2007
(51) Int. Cl.: A61M 5/142, A61M 5/145, A61M 5/315, B23Q 5/40, F16H 25/20, A61M 5/24

(54) **Fördervorrichtung für ein Gerät für die Verabreichung eines Produkts**
Delivery device for an apparatus for dispensing a product
Dispositif pour un appareil pour l'administration d'un produit

(43) Veröffentlichungstag der Anmeldung: 14.01.2009
(73) Patentinhaber: F.HOFFMANN-LA ROCHE AG, 4070 Basel (CH); Roche Diagnostics GmbH, 68305 Mannheim (DE)
(72) Erfinder: Feldmann, Peter, 3326 Krauchthal (CH); Hänggi, Roger, 4208 Nunningen (CH); Niklaus, Hanspeter, 4853 Riken (CH)
(74) Vertreter: Schwabe - Sandmair - Marx

(56) Entgegenhaltungen:
- WO-A-2005/094921
- US-A- 1 751 139
- US-A- 1 905 569
- US-A- 2 627 270
- US-A- 4 769 009
- US-A- 6 117 142

## Beschreibung

Die Erfindung betrifft eine Fördervorrichtung für ein Gerät für die Verabreichung eines Produkts. Bei dem Gerät kann es sich insbesondere um ein Infusionsgerät oder Injektionsgerät für die Verabreichung eines Medikaments, eines Diagnostiziermittels oder grundsätzlich auch jedes anderen dem Menschen oder Tieren verabreichbaren Produkts handeln. Eine bevorzugte Anwendung der Erfindung ist die Selbstverabreichung von Medikamenten, insbesondere die Verabreichung von Insulin in der Diabetestherapie.

Infusionsgeräte und auch Injektionsgeräte bedienen sich für eine genau dosierte Verabreichung des Produkts in vielen Fällen eines Gewindetriebs, um ein Drehmoment eines Antriebsmotors im Gewindeeingriff in eine axiale Translationsbewegung eines Förderglieds umzuwandeln und durch die axiale Bewegung das Produkt aus einem Reservoir des Infusions- oder Injektionsgeräts auszuschütten. Nach Entleerung des Reservoirs muss das Förderglied für eine Wiederverwendung im Gewindeeingriff in eine Ausgangsposition zurückgedreht werden, was entweder mühsam manuell geschehen kann oder eines der Richtung nach umkehrbaren Antriebsmotors bedarf, der für das Zurückdrehen Energie verbraucht.

Die WO 2005/094921 A1 schlägt einen Gewindetrieb mit zwei im Gewindeeingriff befindlichen Fördergliedern vor, von denen eines im Bereich seines Gewindes geschlitzt und daher radial nachgiebig ist. Das betreffende Förderglied kann daher axial, ohne Drehbewegung, bis gegen einen Anschlag in den Gewindeeingriff mit dem anderen Förderglied gestoßen werden. Um trotz der radialen Nachgiebigkeit in der Anschlagposition den Gewindeeingriff herstellen zu können, muss das im Bereich seines Gewindes nachgiebige Förderglied in der Anschlagposition jedoch um die Gewindeachse verdreht werden. Die Herstellung des Gewindeeingriffs erfordert am Ende des axialen Einstoßens einen besonderen Handgriff und daher besondere Aufmerksamkeit und auch Geschicklichkeit. Dies ist insbesondere für Anwendungen problematisch, in denen der Verwender sich das Produkt selbst verabreicht, wie beispielsweise in der Diabetestherapie. Die WO 2005/09492 A1 beschreibt auch noch einen modifizierten Mechanismus, bei dem der Gewindeeingriff sich bei dem axialen Einstoßen des Förderglieds automatisch ergibt. In der Modifikation wird ein nachgiebiger Gewindebereich mittels radial zur Gewindeachse abragenden, elastisch biegbaren und daher entsprechend schlanken Segmenten geschaffen, die gemeinsam das Gewinde des Förderglieds bilden. Bei dem Einstoßen des Förderglieds gleiten die biegbaren Segmente federnd über das Gewinde des anderen Förderglieds. Im Gewindeeingriff drücken die das nachgiebig Gewinde bildenden Segmente daher stets mit ihrer auf Biegeelastizität beruhenden Federkraft in die Gewindegänge des anderen Förderglieds. Für den Antrieb wird entsprechend viel Energie benötigt.

Die US 4 769 009 offenbart eine Fördervorrichtung gemäß der Präambel von Anspruch 1.

Es ist eine Aufgabe der Erfindung, eine Fördervorrichtung der genannten Art mit Fördergliedern zu schaffen, die einfach mittels einer relativ zueinander auszuführenden Axialbewegung in einen leichtgängigen Gewindeeingriff miteinander gebracht werden können.

Diese Aufgabe wird durch die in Anspruch 1 definierte Fördervorrichtung gelöst.

Die Erfindung hat eine Fördervorrichtung für ein Gerät für die Verabreichung eines dem Menschen oder Tieren verabreichbaren Produkts zum Gegenstand, das eine Basis, ein relativ zu der Basis bewegliches erstes Förderglied und ein relativ zu der Basis bewegliches zweites Förderglied aufweist. Das erste Förderglied ist mit einem ersten Gewinde und dass zweite Förderglied mit einem zweiten Gewinde versehen. Die Gewinde sind bei der Verabreichung des Produkts miteinander in einem Gewindeeingriff, so dass die Förderglieder miteinander einen Gewindetrieb bilden. Das zweite Gewinde erstreckt sich über einen in Bezug auf seine Gewindeachse radial nachgiebigen Axialabschnitt des zweiten Förderglieds, so dass eines der Förderglieder in das andere axial in eine Überlappung der Gewinde geschoben werden kann und es für die Herstellung der axialen Überlappung keiner relativen Drehbewegung zwischen den Fördergliedern bedarf. Die Basis bildet ein Gestell für das erste und das zweite Förderglied. Die Basis kann ein Gehäuse des Verabreichungsgeräts umfassen, in dem weitere Komponenten des Verabreichungsgeräts, beispielsweise ein Reservoir für das Produkt, aufgenommen sind. Die Basis kann jedoch auch ein Anbau- oder vorzugsweise Einbauteil sein, das an oder vorzugsweise in einem solchen Gehäuse des Verabreichungsgeräts angeordnet oder für eine derartige Anordnung vorgesehen ist.

Nach der Erfindung umfasst die Fördervorrichtung zusätzlich zu den genannten Komponenten ein Sperrglied, das relativ zu dem zweiten Förderglied oder relativ zu dem das zweite Förderglied in eine Sperrposition bewegbar ist, in der das Sperrglied das zweite Gewinde durch radiale Versteifung des nachgiebigen Axialabschnitts im Gewindeeingriff mit dem Gewinde des ersten Förderglieds hält. Das Sperrglied verhindert in der Sperrposition, dass sich das zweite Gewinde wegen der Nachgiebigkeit des Axialabschnitts unter axialer Last aus dem Gewindeeingriff bewegen kann. Wegen der Versteifung des nachgiebigen Axialabschnitts und somit des zweiten Gewindes mittels eines Sperrglieds, das zusätzlich vorgesehen ist, kann der Gewindeeingriff der Förderglieder auf einfache Weise und zumindest dem Grunde nach ohne Relativdrehung hergestellt werden. Es müssen auch keine filigranen Biegezungen geformt werden, die im Gewindeeingriff zwangsläufig klemmen, da nicht eine rückstellende Elastizitätskraft des Förderglieds den Gewindeeingriff gewährleisten muss, sondern für die Aussteifung des radial nachgiebigen Gewindes das zusätzliche Sperrglied dient. In Abhängigkeit von der Geometrie des zweiten Förderglieds im Bereich des zweiten Gewindes kann das Sperrglied in der Sperrposition das zweite Gewinde auch mit einem geringen radialen Pressdruck in den Gewindeeingriff drücken, wobei der Pressdruck vorzugsweise nur zwischen dem Sperrglied und dem zweiten Förderglied, jedoch nicht zwischen den beiden Gewinden besteht. Das Sperrglied und das zweite Förderglied können Punkt-, Linien- oder Flächenkontakt haben, wobei die einander kontaktierenden Punkte, Linien oder Flächen in Umfangsrichtung um die Gewindeachse zumindest im Wesentlichen um 180° zueinander versetzt sind.

Das Sperrglied wird für die Herstellung des Gewindeeingriffs nicht nur relativ zu dem zweiten Förderglied in die Sperrposition bewegt, sondern gleichzeitig auch relativ zu der Basis und dem ersten Förderglied. Für eine alternative Ausführung ist jedoch auch denkbar, dass das Sperrglied vor der Herstellung des Gewindeeingriffs bereits die Sperrposition einnimmt und im letzten Moment der relativen Einführbewegung zwischen den Fördergliedern in einen Eingriff mit dem zweiten Förderglied gelangt, in dem es dessen nachgiebigen Axialabschnitt zumindest im Bereich des zweiten Gewindes versteift, um das zweite Gewinde unter Last in dem Gewindeeingriff mit dem ersten Gewinde gegen Nachgeben zu sichern. Der Eingriff ist in solch einer alternativen Ausführung ferner so gestaltet, dass das zweite Förderglied bei seiner Förderbewegung das Sperrglied relativ zum ersten Förderglied mitnimmt. Das Sperrglied und das zweite Förderglied können hierfür beispielsweise miteinander verhaken.

Das zweite Förderglied holt das Sperrglied sozusagen aus der Sperrposition ab, damit das Sperrglied während der Verabreichung für die Aussteifung im Bereich des Gewindes sorgt. Im Ergebnis ist das Sperrglied somit zumindest relativ zu dem zweiten Förderglied bis in die Sperrposition bewegbar, was auch den Fall einschließt, dass sich nicht das Sperrglied, sondern das zweite Förderglied relativ zu dem ersten Förderglied und vorzugsweise auch der Basis bewegt, bis der das Gewinde aussteifende Eingriff mit dem Sperrglied hergestellt ist. Für die Versteifung des zweiten Gewindes werden zumindest das Sperrglied und das zweite Förderglied relativ zueinander in die Sperrposition bewegt. In der bevorzugten ersten Variante wird das Sperrglied hierfür relativ zu dem zweiten Förderglied und auch relativ zu dem ersten Förderglied und der Basis bewegt. In der zweiten Variante wird das Sperrglied zumindest während der Verabreichung des Produkts relativ zu dem ersten Förderglied und der Basis bewegt. Das Sperrglied ist somit relativ zu dem ersten Förderglied und der Basis beweglich. Um die Funktion des Versteifens erfüllen zu können, ist das Sperrglied an den nachgiebigen Axialabschnitt des zweiten Förderglieds, zumindest an diesen Axialabschnitt im Bereich des Gewindes angepasst geformt.

Erfindungsgemäß ist das Sperrglied relativ zu dem zweiten Förderglied oder das zweite Förderglied relativ zu dem Sperrglied in axialer Richtung, d.h. längs der Gewindeachse, vorzugsweise zentrisch zum zweiten Gewinde auf der Gewindeachse, in die Sperrposition bewegbar. Vorteilhafterweise führt das Sperrglied die Bewegung in die Sperrposition sowohl relativ zu dem zweiten Förderglied als auch zu dem ersten Förderglied und vorzugsweise auch zu der Basis aus. Das Sperrglied und das zweite Förderglied werden vorzugsweise in die gleiche axiale Richtung relativ zu dem ersten Förderglied bewegt, das zweite Förderglied in die axiale Überlappung der Gewinde und das Sperrglied in die Sperrposition.

Das zweite Förderglied nimmt das Sperrglied vorzugsweise bei seiner im Verlaufe der Verabreichung des Produkts relativ zu dem ersten Förderglied ausgeführten Förderbewegung mit, so dass das Sperrglied stets für die Versteifung im Bereich des zweiten Gewindes sorgt. Vorzugsweise lagert das zweite Förderglied das Sperrglied unmittelbar.

Das Sperrglied ist in der Sperrposition in bevorzugten Ausführungen relativ zu dem zweiten Förderglied axial fixiert. Die Fixierung kann grundsätzlich zwar kraftschlüssig, auch rein kraftschlüssig sein, bevorzugt ist sie jedoch formschlüssig oder umfasst einen formschlüssigen Eingriff zumindest. Die Fixierung kann insbesondere mittels eines Rasteingriffs gebildet sein, der vorzugsweise unmittelbar zwischen dem Sperrglied und dem zweiten Förderglied besteht.

In einer Weiterbildung ist das Sperrglied aus einem lösbaren Fixiereingriff, der rein kraftschlüssig oder rein formschlüssig oder form- und kraftschlüssig, vorzugsweise als Rasteingriff, gebildet sein kann, relativ zu dem zweiten Förderglied aus einer Ausgangsposition in die Sperrposition bewegbar. Der das Sperrglied in der Ausgangsposition haltende Fixiereingriff kann insbesondere unmittelbar zwischen dem Sperrglied und dem zweiten Förderglied bestehen.

Das zweite Förderglied und das Sperrglied bilden gemeinsam eine Kolbenstange , für die Förderrung des Produkts mittels eines axial im Reservoir beweglichen Kolbens. In derartigen Ausführungen ist es ferner vorteilhaft, wenn das Sperrglied ein mit dem Kolben verbundenes oder vorzugsweise nur zugewandtes vorderes Ende der Kolbenstange bildet.

Das Sperrglied und das zweite Förderglied bilden miteinander ein Teleskop. Bevorzugt wird das Teleskop für die Aussteifung des zweiten Gewindes zusammengefahren, d.h. in seiner axialen Länge verkürzt. Das Sperrglied ragt vorzugsweise in das zweite Förderglied, eine Umkehrung dieser Verhältnisse ist grundsätzlich jedoch möglich, in Abhängigkeit von der Ausbildung des zweiten Gewindes als Innengewinde oder vorzugsweise Außengewinde.

Die axiale Nachgiebigkeit des mit dem zweiten Gewinde versehenen Axialabschnitts kann insbesondere mittels einer oder mehreren axial erstreckten Durchbrechung(en) des zweiten Förderglieds im betreffenden Axialabschnitt erhalten werden. Die Durchbrechungen erstrecken sich vorteilhafterweise bis zu einem axialen Ende des zweiten Förderglieds, so dass das zweite Förderglied in seinem nachgiebigen Axialabschnitt mehrere axial erstreckte Arme aufweist, die von dem betreffenden axialen Ende entfernt vorzugsweise in einem vereinigenden Abschnitt des zweiten Förderglieds zusammenlaufen. Wenigstens einer der Arme, vorzugsweise mehrere der Arme und bevorzugt alle Arme übernimmt oder übernehmen gemeinsam die Gewindefunktion, indem der jeweilige Arm an einer Umfangsfläche ein Bogensegment des Gewindes bildet. Grundsätzlich würde es auch genügen, wenn das zweite Förderglied nur einen Arm aufweist, der radial nachgiebig ist und ein Gewindesegment bildet. Bevorzugter weist das zweite Förderglied mehrere Arme auf, beispielsweise vier Arme, die in einer Ansicht auf das hintere Ende des zweiten Förderglieds durch beispielsweise eine kreuzförmige Schlitzung dieses Förderglieds erhalten werden. In besonders bevorzugten Ausführungen weist das zweite Förderglied in seinem nachgiebigen Axialabschnitt genau zwei Arme auf, die durch eine einfache, axial erstreckte Schlitzung erhalten werden. Die Arme sind axial erstreckte Biegearme, sind also von ihrem jeweiligen axialen Befestigungsende aus nach radial innen oder vorzugsweise nach radial außen in den Gewindeeingriff biegbar, so dass die mit dem jeweiligen Gewindesegment versehene Umfangsfläche des jeweiligen Arms quer zu der Richtung weist, in die dieses Gewindesegment in den Gewindeeingriff bewegt wird.

Obgleich es genügen würde, wenn das zweite Gewinde nur einen einzigen Gewindegang aufweist oder sich sogar nur über einen Winkel von weniger als 360° um die Gewindeachse erstreckt, wird es jedoch bevorzugt, wenn das zweite Gewinde mehr als einen vollständigen Gewindegang, vorzugsweise mehrere vollständige Gewindegänge umfasst. Andererseits umfasst es vorteilhafterweise deutlich weniger Gewindegänge als das erste Gewinde, ist in bevorzugten Ausführungen also axial deutlich kürzer als dieses. So weist das erste Gewinde vorzugsweise vielfach mehr Gewindegänge als das zweite Gewinde auf.

Das zweite Gewinde, genauer gesagt dessen Umfangshüllfläche, ist im ausgesteiften Zustand, wenn der nachgiebige Axialabschnitt des zweiten Förderglieds im Bereich des zweiten Gewindes an dem Sperrglied anliegt, vorzugsweise zylindrisch. Auch dies trägt zu einem leichtgängigen, sauberen Gewindeeingriff bei. In einer bevorzugten Variante entspricht dies auch dem unbelasteten Zustand des nachgiebigen Axialabschnitts. In anderen Varianten kann der nachgiebige Axialabschnitt aber auch beispielsweise gerade oder gerkrümmt zur Gewindeachse geneigt sein. Auch für diese anderen Varianten gilt jedoch vorteilhafterweise, dass die Umfangshüllfläche des zweiten Gewindes mit in der Sperrposition befindlichem Sperrglied zylindrisch ist, also mittels des Sperrglieds in die Zylinderform gebracht wird.

Das zweite Förderglied und das Sperrglied sind in bevorzugten Ausführungen nur für einen einmaligen Gebrauch konzipiert und werden bei einem Austausch des Reservoirs oder einem Wiederauffüllen gegen ein neues zweites Förderglied und ein neues Sperrglied ausgetauscht. Das erste Förderglied und die Basis werden hingegen bevorzugt mehrfach benutzt. In solch einem Konzept kann es von Vorteil sein, wenn das zweite Förderglied aus einem weicheren Material als das erste Förderglied oder zumindest das zweite Gewinde mit weicherem Material als das erste Gewinde geformt ist. Die Förderglieder und das Sperrglied sind in bevorzugten Ausführungen je in einem Stück geformt, beispielsweise im Spritzguss aus Kunststoff. Eines der Förderglieder oder beide Förderglieder oder das Sperrglied können grundsätzlich jedoch auch aus mehreren separat geformten Teilen bestehen, die jeweils so miteinander verbunden sind, dass sie die für die Herstellung des Gewindeeingriffs und die bei der Förderung des Produkts erforderlichen Bewegungen jeweils als Einheit ausführen.

Die Erfindung ist durch Anspruch 1 definiert.

Vorteilhafte Merkmale werden in den Unteransprüchen und in deren Kombination beschrieben.

Nachfolgend wird ein Ausführungsbeispiel der Erfindung anhand von Figuren erläutert. Es zeigen:
- Figur 1: eine Fördervorrichtung in einem Ausgangszustand vor dem Zusammenbau eines zwei Förderglieder aufweisenden Gewindetriebs,
- Figur 2: die Fördervorrichtung nach dem Zusammenbau,
- Figur 3: die im Ausgangszustand befindliche Fördervorrichtung in einem Längsschnitt,
- Figur 4: die im Ausgangszustand befindliche Fördervorrichtung in einer teilweisen Sicht und einem teilweisen Längsschnitt,
- Figur 5: eines der Förderglieder in einem Längsschnitt und
- Figur 6: ein Sperrglied der Fördervorrichtung in einem Längsschnitt.

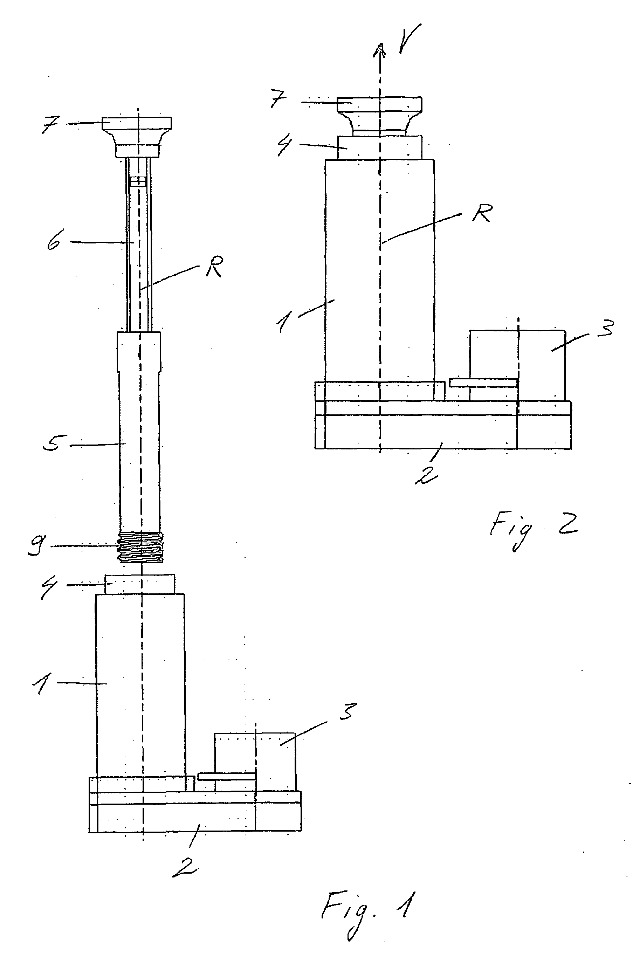

Figur 1 zeigt eine Fördervorrichtung eines Geräts für die Verabreichung eines flüssigen Produkts. Im Ausführungsbeispiel bildet eine auf oder unter der Kleidung am Körper tragbare Infusionspumpe für eine subkutane Verabreichung eines flüssigen Medikaments, beispielsweise Insulin, das Gerät. Die in Figur 1 dargestellte Fördervorrichtung kann insbesondere in einem Gehäuse solch eines Geräts angeordnet sein. Sie dient der Förderung des Produkts, das in einem Reservoir enthalten ist. In dem Reservoir ist ein Kolben bewegbar angeordnet. Durch eine Vortrieb des Kolbens in eine axiale Richtung auf einen Auslass des Reservoirs zu wird das Produkt für die Verabreichung gefördert. Die Fördervorrichtung dient dem Vortrieb solch eines Kolbens.

Die Fördervorrichtung umfasst Lagerstrukturen 1 und 2, die gemeinsam eine Basis 1, 2 für die Lagerung und Abstützung weiterer Komponenten der Fördervorrichtung bilden. Die Fördervorrichtung umfasst ferner einen motorischen Antrieb 3, ein erstes Förderglied 4, ein zweites Förderglied 5 und ein Sperrglied 6. Das erste Förderglied 4 ist relativ zu der Basis 1, 2 um eine Rotationsachse R drehbar und axial fixiert. Die axiale Fixierung relativ zu der Basis 1, 2 kann innerhalb von Fertigungstoleranzen absolut sein. In einer Variante können kleinere axiale Hubbewegungen längs der Rotationsachse R möglich sein, beispielsweise um eine auf das Förderglied 4 bei der Förderung des Produkts wirkende Axialkraft zu messen. Das zweite Förderglied 5 ist in Figur 1 losgelöst vom ersten Förderglied 4 vor einem Zusammenbau der Fördervorrichtung dargestellt.

Figur 2 zeigt die Fördervorrichtung im zusammengebauten Zustand. Das zweite Förderglied 5 ragt in das erste Förderglied 4. Die Förderglieder 4 und 5 sind im zusammengebauten Zustand miteinander in einem Gewindeeingriff, dessen Gewindeachse die Rotationsachse R ist, die im folgenden daher als Gewindeachse R bezeichnet wird. Der Antrieb 3 ist mittels eines Getriebes im Bereich der Lagerstruktur 2 mit dem ersten Förderglied 4 gekoppelt. Das Getriebe kann insbesondere ein Zahnradgetriebe sein. Der Antrieb 3 ist ein Drehmotor, beispielsweise elektrischer Schrittmotor, mit einer zu der Gewindeachse R parallelen Motorachse. Das Getriebe kann dementsprechend ein einfaches Stirnradgetriebe sein. Das erste Förderglied 4 ist drehsteif mit einem Getrieberad verbunden, was auch den Fall einschließt, dass solch ein Getrieberad in einem Stück mit dem Förderglied 4 geformt sein kann.

Das zweite Förderglied 5 und das Sperrglied 6 bilden gemeinsam eine Kolbenstange, die bei einem Drehantrieb des Förderglieds 4 in einem Gewindeeingriff der Förderglieder 4 und 5 relativ zu der Basis 1, 2 und dem Förderglied 4 axial bewegt wird. Bei einer axialen Bewegung in eine Vortriebsrichtung V drückt die Kolbenstange 5, 6 gegen den Kolben, so dass dieser ebenfalls in die Vortriebsrichtung V auf den Auslass des Reservoirs zu bewegt und entsprechend der Vortriebsbewegung Produkt ausgeschüttet wird. Das Sperrglied 6 weist an seinem in Vortriebsrichtung V vorderen Ende einen Stempel 7 auf, mit dem es in die Vortriebsrichtung V gegen den Kolben drückt und diesen dadurch vortreibt. Zwischen der Kolbenstange 5, 6 und dem Kolben besteht nur Druckkontakt in die Vortriebsrichtung V. Eine Verbindung in die Gegenrichtung besteht nicht, so dass die Kolbenstange 5, 6 durch eine Bewegung relativ zu dem Kolben entgegen der Vortriebsrichtung V von dem Kolben auf einfache Weise gelöst werden kann.

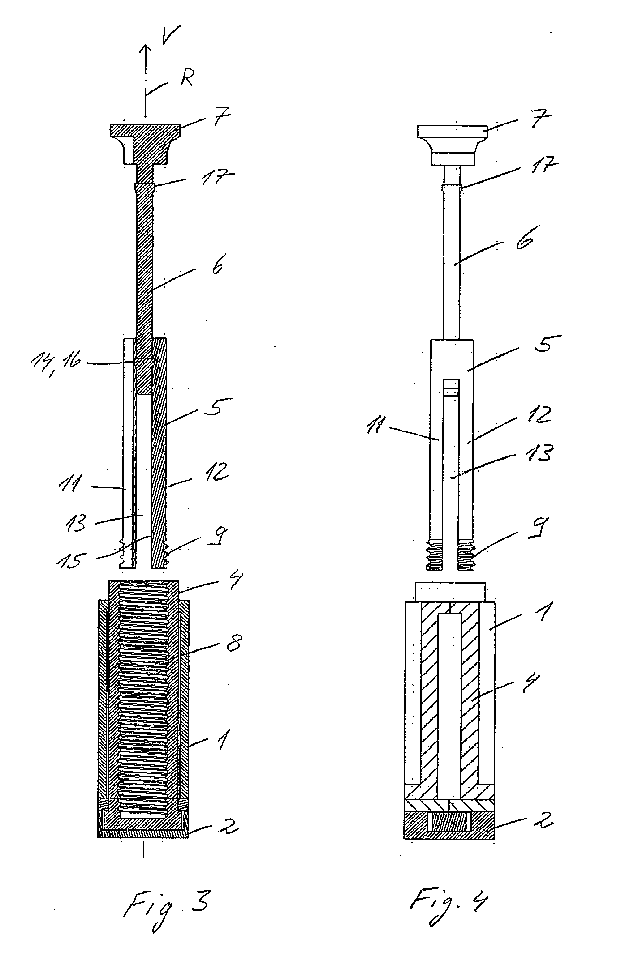

Figur 3 zeigt die Fördervorrichtung in einem Längsschnitt in einer zu der Sichtebene der Figur 1 im rechten Winkel stehenden Schnittebene. Die Gewindeachse R erstreckt sich in der Schnittebene. In Figur 4 ist die Fördervorrichtung in einer zu der Schnittebene der Figur 3 parallel versetzten Sicht- und Schnittebene dargestellt, die sich in einem Abstand von der Kolbenstange 5, 6 durch das Förderglied 4 erstreckt. Die Fördervorrichtung befindet sich in den Figuren 3 und 4 im noch nicht zusammengebauten Zustand, wie in Figur 1.

Die Kolbenstange 5, 6, nämlich die Kombination aus Förderglied 5 und Sperrglied 6, ist von dem in Figur 3 dargestellten Ausgangszustand ausgehend gegen die Vortriebsrichtung V axial in das Förderglied 4 bis in eine axiale Anschlagposition einführbar. Die in Figur 2 dargestellte Anschlagposition wird durch Anschlagkontakt des Förderglieds 5 an einem Anschlag bestimmt, den im Ausführungsbeispiel das Förderglied 4 bildet, in Abwandlungen jedoch auch beispielsweise die Basis 1, 2, beispielsweise die Lagerstruktur 2, bilden könnte.

Das Förderglied 4 ist eine Hülsenstruktur mit einem Hülsenabschnitt und einem Boden an einem axial hinteren Ende. Der Hülsenabschnitt erstreckt sich über nahezu die gesamte axiale Länge des Förderglieds 4. An einer Mantelinnenfläche des Hülsenabschnitts ist ein erstes Gewinde 8 geformt. Das Gewinde 8 erstreckt sich über die gesamte Länge des Hülsenabschnitts des Förderglieds 4. Das Förderglied 4 könnte auch ohne den Boden axial durchgehend als Hülse gebildet sein. In derartigen Modifikationen könnte sich das Gewinde 8 axial durchgehend über die gesamte Länge des Förderglieds 4 erstrecken.

Das Förderglied 5 weist an seinem hinteren Ende am äußeren Umfang ein zweites Gewinde 9 auf, das für den Gewindeeingriff mit dem Gewinde 8 angepasst an dieses als Außengewinde geformt ist. Das Gewinde 9 erstreckt sich nur über einen kleineren Teil der axialen Länge des Gewindes 8, umfasst aber gleichwohl mehrere Gewindegänge für einen klemmfreien und daher leichtgängigen, aber dennoch festen Gewindeeingriff zu gewährleisten. Das Förderglied 5 ist im Wesentlichen ebenfalls hülsenförmig und umfasst in einem vorderen Bereich einen in Umfangsrichtung um die Gewindeachse R in sich zurücklaufenden Hülsenabschnitt und von diesem entgegen der Vortriebsrichtung V nach hinten axial abragende Arme 11 und 12, an denen das Gewinde 9 gebildet ist. Die Arme 11 und 12 bilden an ihrem äußeren Umfang je ein Bogensegment des Gewindes 9. Jedes der Bodensegmente erstreckt sich in Umfangsrichtung um die Gewindeachse R über einen Winkel von weniger als 180°. Das Gewinde 9 erstreckt sich an den Armen 11 und 12 bis jeweils zum axialen hinteren Ende. Die Arme 11 und 12 werden auf ihren beiden Seiten durch je eine Durchbrechung 13 voneinander separiert, die sich von dem Hülsenabschnitt am vorderen Ende des Förderglieds 5 durchgehend bis zu dem hinteren Ende des Förderglieds 5 erstreckt. Die Arme 11 und 12 sind elastisch aufeinander zu in die Durchbrechungen 13 hinein biegbar, solange der Raum zwischen den Armen 11 und 12 frei ist. Das Förderglied 5 entspricht der Form nach einer zylindrischen Hülse, die von ihrem hinteren Ende her bis zu dem vorderen Hülsenabschnitt durchgehend geschlitzt ist, so dass die Durchbrechungen 13 jeweils die Form von geraden axialen Schlitzen haben. Die Arme 11 und 12 bilden einen nachgiebigen Axialabschnitt des Förderglieds 5 und ein radial nachgiebiges Gewinde 9.

Das Sperrglied 6 nimmt in dem in den Figuren 1, 3 und 4 dargestellten Ausgangszustand der Fördervorrichtung relativ zu dem Förderglied 5 eine axiale Ausgangsposition ein. In der Ausgangsposition ragt es nur mit einem axial hinteren Endabschnitt in den vorderen Hülsenabschnitt des Förderglieds 5. Das Sperrglied 6 ist in der Ausgangsposition lösbar in axialer Richtung relativ zu dem Förderglied 5 fixiert. Im Ausführungsbeispiel ist der Fixiereingriff unmittelbar zwischen dem Förderglied 5 und dem Sperrglied 6 als Rasteingriff gebildet. Im Rasteingriff sind eine erste Rasteinrichtung 14 des Förderglieds 5 und eine erste Rastgegeneinrichtung 16 des Sperrglieds 6 miteinander verrastet. Das Sperrglied 6 kann relativ zum Förderglied 5 aus diesem Fixiereingriff gegen eine rückstellende Elastizitätskraft nur entgegen der Vortriebsrichtung V bewegt werden, eine Relativbewegung in die Vortriebsrichtung V wird durch den Fixiereingriff verhindert. Die Rasteinrichtung 14 ist an einer Mantelinnenfläche des Förderglieds 5 in Form von Vertiefungen gebildet. Im Ausführungsbeispiel sind in Umfangsrichtung um 180° zueinander versetzt zwei solche Rastmittel 14 geformt. Die Rastgegeneinrichtung 16 ist entsprechend am äußeren Umfang des Sperrglieds 6 als radial abragende Rastnocken geformt. Im Ausführungsbeispiel sind entsprechend der Anzahl der Rastmittel 14 angepasst geformte und am Sperrglied 6 angeordnete Rastgegenmittel 16 geformt. Anstatt die Rastmittel 14 je als Vertiefung und die Rastgegenmittel 16 je als Erhebung bzw. Nocken zu formen, könnten auch die Rastmittel 14 als Erhebungen bzw. Nocken und die Rastgegenmittel 16 als Vertiefungen geformt sein, ebenso sind Mischformen denkbar. Die Rasteinrichtung 14 und Rastgegeneinrichtung 16 könnten je auch nur aus einem einzigen Rastmittel 14 und entsprechend Rastgegenmittel 16 bestehen, eine gleichmäßige Verteilung mehrerer Rastmittel und Rastgegenmittel 14 und 16 in Umfangsrichtung um die Gewindeachse R wird jedoch bevorzugt.

Das Sperrglied 6 ragt in der Ausgangsposition axial nur so weit in das Förderglied 5 hinein, dass es die Biegebewegung der Arme 11 und 12 aufeinander zu nicht behindert, zumindest die Bewegung der Bogensegmente des Gewindes 9 aufeinander zu nicht verhindert. Aus der Ausgangsposition ist das Sperrglied 6 relativ zu dem Förderglied 5 axial gegen die Vortriebsrichtung V bis in eine Sperrposition bewegbar, in der es in den in der Ausgangsposition noch freien Raum zwischen den Bogensegmenten des Gewindes 9 hineinragt und diesen Raum radial zwischen den Bogensegmenten des Gewindes 9 ausfüllt. Wenn das Sperrglied 6 relativ zu dem Förderglied 5 die Sperrposition einnimmt, können sich die Bogensegmente des Gewindes 9 somit nicht mehr quer zu einer gedachten Umfangshüllfläche des Gewindes 9 aufeinander zu bewegen, so dass der in der Ausgangsposition des Sperrglieds 6 noch nachgiebige, das Gewinde 9 aufweisende Axialabschnitt versteift ist und mit dem in der Sperrposition befindlichen Sperrglied 6 einen steifen Gewindeabschnitt bildet.

Das Sperrglied 6 ist in der Sperrposition relativ zu dem Förderglied 5 axial fixiert. Auch dieser weitere Fixiereingriff ist unmittelbar zwischen dem Förderglied 5 und dem Sperrglied 6 gebildet. Er stellt sich automatisch ein, wenn das Sperrglied 6 aus der Ausgangsposition tiefer in das Förderglied 5 bis in die Sperrposition gedrückt wird. Dieser zweite Fixiereingriff ist ebenfalls als Rasteingriff gebildet, nämlich zwischen einer zweiten Rasteinrichtung 15 des Förderglieds 5 und der ersten Rastgegeneinrichtung 16 des Sperrglieds 6. Entsprechend der Formung der Rastgegeneinrichtung 16 als ein oder mehrere Nocken ist auch die zweite Rasteinrichtung 15 als eine oder mehrere Vertiefung(en) geformt, im Ausführungsbeispiel mit jeweils einer Vertiefung an einer Mantelinnenfläche jedes der Arme 11 und 12. Um den Fixiereingriff in der Sperrposition des Sperrglieds 6 zu festigen, ist auch das Sperrglied 6 mit einer zweiten Rastgegeneinrichtung 17 versehen, die in der Sperrposition in einen Fixiereingriff, im Ausführungsbeispiel Rasteingriff, mit der ersten Rasteinrichtung 14 des Förderglieds 5 gelangt. Das zu der Rasteinrichtung 14 und der Rastgegeneinrichtung 16 für den Fixiereingriff in der Ausgangsposition Gesagte gilt gleichermaßen auch für die zweite Rasteinrichtung 15 und die zweite Rastgegeneinrichtung 17 und den jeweiligen Fixiereingriff in der Sperrposition des Sperrglieds 6.

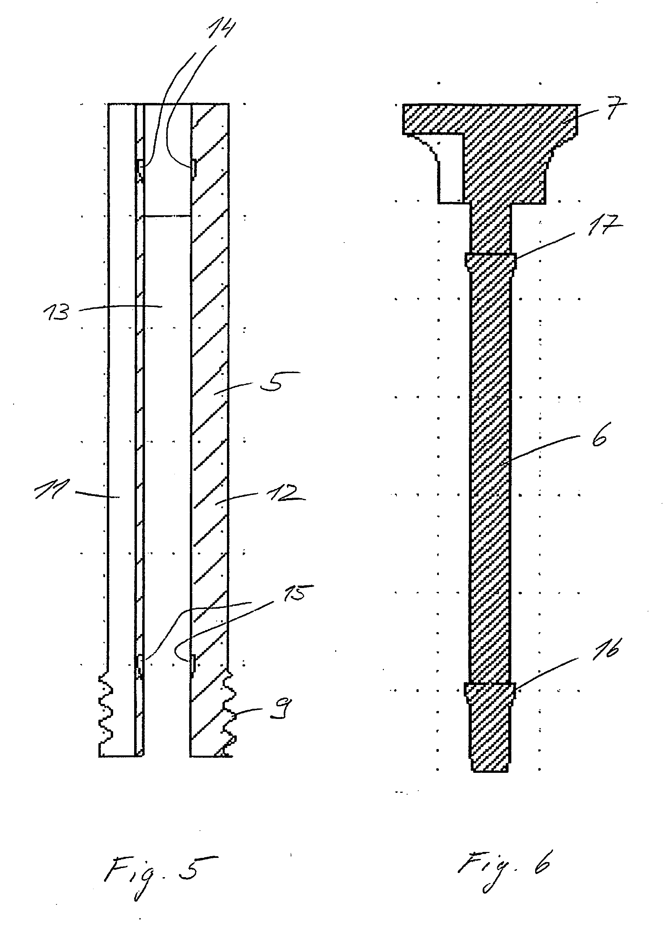

Die Rast- und Rastgegeneinrichtungen 14 bis 17 sind deutlicher als in Figur 3 in den Figuren 5 und 6 zu erkennen, die das Förderglied 5 und das Sperrglied 6 jeweils einzeln und im gleichen Schnitt wie die Figur 3 zeigen.

Das Sperrglied 6 ist mit dem Förderglied 5 axial beweglich, aber in Bezug auf die Gewindeachse R verdrehgesichert verbunden. Das Förderglied 5 bildet für das Sperrglied 6 eine axiale Führungsbahn. Das Förderglied 5 ist relativ zu der Basis 1, 2 axial längs der Gewindeachse R beweglich und wird an Drehbewegungen um die Gewindeachse R durch einen entsprechenden Führungseingriff gehindert, beispielsweise einen Führungseingriff der Basis 1, 2. Im Eingriff der Gewinde 8 und 9 wird bei einem Drehantrieb des Förderglieds 4 daher das Förderglied 5 und mit diesem gemeinsam das Sperrglied 6 axial in die Vortriebsrichtung V bewegt und schiebt bei seiner Axialbewegung den Kolben im Reservoir ebenfalls in die Vortriebsrichtung V vor, um Produkt auszuschütten. Wenn das Reservoir entleert ist, ragt das Förderglied 5 soweit aus dem Förderglied 4 heraus, dass nur noch die Gewinde 8 und 9 in axialer Überlappung miteinander sind.

Nach Entleerung des Reservoirs kann das Förderglied 5 rasch und mit geringem Energieaufwand mittels des Antriebs 3 und des Förderglieds 4 vollständig aus dem Gewindeeingriff bewegt werden, indem das Förderglied 4 um einige wenige Umdrehungen, deren Anzahl der Anzahl der Gewindegänge des Gewindes 9 entspricht, weitergedreht wird. Das Förderglied 5 und das Sperrglied 6 können gegen ein neues Förderglied 5 und ein neues Sperrglied 6 ausgetauscht werden, falls sie als Wegwerfteile konzipiert sind, oder gegebenenfalls wieder verwendet werden, wofür allerdings die Fixierung des Sperrglieds 6 in der Sperrposition lösbar gestaltet sein muss.

Im Falle des bevorzugten Austausches greift der Verwender nach Ausstoss der alten Kolbenstange 5, 6 die neue Kombination aus Förderglied 5 und Sperrglied 6 mit dem in der Ausgangsposition am Förderglied 5 fixierten neuen Sperrglied 6 und führt das Förderglied 5 axial gegen die Vortriebsrichtung V in das Förderglied 4 ein. Die Einführbewegung ist aufgrund der biegeelastischen Arme 11 und 12 auf einfache Weise möglich. Der Verwender muss die Kombination aus Förderglied 5 und Sperrglied 6 lediglich wie ein Stössel mit leichtem Druck in das Förderglied 4 bis in die Anschlagposition drücken. Das Gewinde 9 gleitet bei dieser Axialbewegung entsprechend seiner rückstellenden Biegeelastizität mit leichtem radialen Druck an dem Gewinde 8 federnd ab. Sobald das Förderglied 5 die Anschlagposition erreicht hat, drückt der Verwender das Sperrglied 6 gegen die Vortriebsrichtung V axial tiefer in das Förderglied 5 bis es mit diesem in der Sperrposition verrastet. Das Erreichen der Sperrposition wird taktil wahrgenommen, vorzugsweise zusätzlich mittels eines Sensors der Fördervorrichtung detektiert. Vorzugsweise wird die korrekte Positionierung des Sperrglieds 6 dem Verwender mittels einer Anzeige des Geräts auch angezeigt, beispielsweise optisch. Wenn das Sperrglied 6 die Sperrposition einnimmt und die Bogensegmente des Gewindes 9 somit nicht mehr aus dem Gewindeeingriff mit dem Gewinde 8 nach innen gebogen werden können, ist ein fester, axial schlupffreier, aber dennoch leichtgängiger Gewindeeingriff gewährleistet, so dass die Vorrichtung bereit ist für die Ausschüttung des Produkts aus einem wieder gefüllten oder neuen, samt Inhalt und Kolben ausgetauschten Reservoir.

### Bezugszeichen:

- 1: Basis, Lagerstruktur
- 2: Basis, Lagerstruktur
- 3: Antrieb
- 4: erstes Förderglied
- 5: zweites Förderglied
- 6: Sperrglied
- 7: Stempel
- 8: erstes Gewinde
- 9: zweites Gewinde
- 10: -
- 11: Arm
- 12: Arm
- 13: Durchbrechung
- 14: erste Rasteinrichtung
- 15: zweite Rasteinrichtung
- 16: erste Rastgegeneinrichtung
- 17: zweite Rastgegeneinrichtung

- R: Gewindeachse
- V: Vortriebsrichtung

## Patentansprüche

1. Fördervorrichtung für ein Gerät für die Verabreichung eines Produkts, die Fördervorrichtung umfassend
a) eine Basis (1, 2),
b) einen Kolben für die Förderung des Produkts und eine Kolbenstange für einen Vortrieb des Kolbens,
c) ein relativ zu der Basis (1, 2) bewegliches erstes Förderglied (4) mit einem ersten Gewinde (8),
d) ein relativ zu der Basis (1, 2) bewegliches zweites Förderglied (5) mit einem zweiten Gewinde (9) für einen Gewindeeingriff mit dem ersten Gewinde (8),
e) wobei sich das zweite Gewinde (9) über einen in Bezug auf seine Gewindeachse (R) radial nachgiebigen Axialabschnitt (11, 12) des zweiten Förderglieds (5) erstreckt, so dass eines der Förderglieder (4, 5) in das andere axial in eine Überlappung der Gewinde (8, 9) einschiebbar ist,
f) und ein relativ zu der Basis (1, 2) bewegliches Sperrglied (6), wobei das zweite Förderglied (5) und das Sperrglied (6) zusammen ein Teleskop bilden und eines aus Sperrglied (6) und zweitem Förderglied (5) relativ zu dem anderen teleskopartig axial in eine Sperrposition bewegbar ist, in der das Sperrglied (6) das zweite Gewinde (9) in dem Gewindeeingriff im Bereich des nachgiebigen Axialabschnitts (11, 12) radial versteift und dadurch den Gewindeeingriff sichert, **dadurch gekennzeichnet, dass**
g) das zweite Förderglied (5) und das Sperrglied (6) zusammen die längs der Gewindeachse (R) bewegliche Kolbenstange (5, 6) bilden.

2. Fördervorrichtung nach dem vorhergehenden Anspruch, **dadurch gekennzeichnet, dass** das Sperrglied (6) oder zweite Förderglied (5) in axialer Richtung in die Sperrposition bewegbar ist, vorzugsweise axial linear geführt wird.

3. Fördervorrichtung nach einem der vorhergehenden Ansprüche, **dadurch gekennzeichnet, dass** das zweite Förderglied (5) das Sperrglied (6) lagert.

4. Fördervorrichtung nach einem der vorhergehenden Ansprüche, **dadurch gekennzeichnet, dass** das Sperrglied (6) in Bezug auf die Gewindeachse (R) verdrehgesichert axial gerade bewegbar geführt wird, vorzugsweise von dem zweiten Förderglied (5).

5. Fördervorrichtung nach einem der vorhergehenden Ansprüche, **dadurch gekennzeichnet, dass** das Sperrglied (6) in der Sperrposition relativ zu dem zweiten Förderglied (5) axial in einem Fixiereingriff fixiert ist, vorzugsweise in einem Rasteingriff.

6. Fördervorrichtung nach einem der vorhergehenden Ansprüche, **dadurch gekennzeichnet, dass** das Sperrglied (6) aus einem lösbaren Fixiereingriff, vorzugsweise Rasteingriff, relativ zu dem zweiten Förderglied (5) in die Sperrposition bewegbar ist.

7. Fördervorrichtung nach einem der zwei vorhergehenden Ansprüche, **dadurch gekennzeichnet, dass** das Sperrglied (6) an einer Umfangsfläche ein oder mehrere Fixiermittel (16, 17) für den Fixiereingriff aufweist, wobei das oder die Fixiermittel (16, 17) vorzugsweise als Vertiefungen oder Erhebungen an der Umfangsfläche geformt sind.

8. Fördervorrichtung nach dem vorhergehenden Anspruch, **dadurch gekennzeichnet, dass** das zweite Förderglied (5) ein oder mehrere Fixiergegenmittel (14, 15) für den Fixiereingriff mit dem oder den Fixiermittel(n) (16, 17) des Sperrglieds (6) aufweist.

9. Fördervorrichtung nach einem der vier vorhergehenden Ansprüche, **dadurch gekennzeichnet, dass** das Sperrglied (6) und das zweite Förderglied (5) in dem Fixiereingriff axial aneinander festgelegt sind.

10. Fördervorrichtung nach einem der vorhergehenden Ansprüche, **dadurch gekennzeichnet, dass** das Sperrglied (6) in der Sperrposition das zweite Gewinde (9) nur formschlüssig radial kraftfrei stützt, solange der Gewindeeingriff axial nicht belastet wird, vorzugsweise nach radial innen stützt, oder in den Gewindeeingriff drückt, vorzugsweise nach radial außen.

11. Fördervorrichtung nach einem der vorhergehenden Ansprüche, **dadurch gekennzeichnet, dass** die Kolbenstange (5, 6) am Kolben axial fixierbar oder lösbar fixiert oder für die Förderung mit dem Kolben vorzugsweise in einem nur in eine Vortriebsrichtung wirksamen axialen Druckkontakt ist.

12. Fördervorrichtung nach einem der vorhergehenden Ansprüche, **dadurch gekennzeichnet, dass** das Sperrglied (6) ein vorderes Ende für eine Kontaktierung des Kolbens bildet.

13. Fördervorrichtung nach einem der vorhergehenden Ansprüche, **dadurch gekennzeichnet, dass** das Sperrglied (6) aus dem zweiten Förderglied (5) ragt und aus einer Ausgangsposition axial tiefer in das zweite Förderglied (5) bis in die Sperrposition bewegbar ist.

14. Fördervorrichtung nach einem der vorhergehenden Ansprüche, **dadurch gekennzeichnet, dass** das zweite Gewinde (9) in dem nachgiebigen Axialabschnitt (11, 12) aus einem oder mehreren Bogensegment(en) besteht, das oder die sich um die Gewindeachse (R) über einen Winkel von höchstens 180° oder jeweils höchstens 180° erstreckt oder erstrecken, wobei im Falle mehrerer Bogensegmente diese in Umfangsrichtung jeweils links und rechts voneinander separiert sind.

15. Fördervorrichtung nach einem der vorhergehenden Ansprüche, **dadurch gekennzeichnet, dass** das zweite Gewinde (9) an einem oder mehreren axial erstreckten Arm(en) (11, 12) des nachgiebigen Axialabschnitts geformt ist.

16. Fördervorrichtung nach dem vorhergehenden Anspruch, **dadurch gekennzeichnet, dass** das zweite Förderglied (5) zwischen in Umfangsrichtung benachbarten Bogensegmenten axial erstreckte Durchbrechungen (13) aufweist, die die Bogensegmente voneinander separieren.

17. Fördervorrichtung nach einem der vorhergehenden Ansprüche, **dadurch gekennzeichnet, dass** das zweite Förderglied (5) zumindest in dem nachgiebigen Axialabschnitt (11, 12) hohl ist.

18. Fördervorrichtung nach einem der vorhergehenden Ansprüche, **dadurch gekennzeichnet, dass** das zweite Gewinde (9) ein Außengewinde ist.

19. Fördervorrichtung nach einem der vorhergehenden Ansprüche, **dadurch gekennzeichnet, dass** das erste Gewinde (8) ein Innengewinde ist.

20. Fördervorrichtung nach einem der vorhergehenden Ansprüche, **dadurch gekennzeichnet, dass** sich das zweite Gewinde (9) nur über einen Teil der axialen Länge des ersten Gewindes (8) erstreckt.

21. Fördervorrichtung nach einem der vorhergehenden Ansprüche, **dadurch gekennzeichnet, dass** eines der Förderglieder (4, 5), vorzugsweise das erste Förderglied (4), relativ zu der Basis (1, 2) um die Gewindeachse (R) drehbar und das andere Förderglied relativ zu der Basis (1, 2) in Bezug auf die Gewindeachse (R) verdrehgesichert axial linear bewegbar ist.

22. Fördervorrichtung nach einem der vorhergehenden Ansprüche, umfassend einen Antrieb (3) für einen automatischen Drehantrieb eines (4) der Förderglieder (4, 5).

## Claims

1. A conveying device for an apparatus for administering a product, said conveying device comprising:
a) a base (1, 2);
b) a piston for conveying the product, and a piston rod for advancing the piston;
c) a first conveying member (4) which can be moved relative to the base (1, 2) and comprises a first thread (8);
d) a second conveying member (5) which can be moved relative to the base (1, 2) and comprises a second thread (9) for a threaded engagement with the first thread (8);
e) wherein the second thread (9) extends over an axial portion (11, 12) of the second conveying member (5) which is radially flexible in relation to its threaded axis (R), such that one of the conveying members (4, 5) can be axially inserted into the other in an overlap of the threads (8, 9);
f) and a blocking member (6) which can be moved relative to the base (1, 2), wherein the second conveying member (5) and the blocking member (6) together form a telescope, and one of the blocking member (6) and the second conveying member (5) can be telescopically axially moved relative to the other into a blocking position in which the blocking member (6) radially stiffens the second thread (9) in the threaded engagement in the region of the flexible axial portion (11, 12) and thus secures the threaded engagement,
**characterised in that**
g) the second conveying member (5) and the blocking member (6) together form the piston rod (5, 6) which can be moved along the threaded axis (R).

2. The conveying device according to the preceding claim, **characterised in that** the blocking member (6) or the second conveying member (5) can be moved in the axial direction into the blocking position and is preferably axially and linearly guided.

3. The conveying device according to any one of the preceding claims, **characterised in that** the second conveying member (5) mounts the blocking member (6).

4. The conveying device according to any one of the preceding claims, **characterised in that** the blocking member (6) is guided, preferably by the second conveying member (5), such that it can be axially and linearly moved but is secured against rotating in relation to the threaded axis (R).

5. The conveying device according to any one of the preceding claims, **characterised in that** in the blocking position, the blocking member (6) is axially fixed relative to the second conveying member (5) in a fixing engagement, preferably in a locking engagement.

6. The conveying device according to any one of the preceding claims, **characterised in that** the blocking member (6) can be moved out of a releasable fixing engagement, preferably a locking engagement, relative to the second conveying member (5) into the blocking position.

7. The conveying device according to any one of the preceding two claims, **characterised in that** a circumferential surface of the blocking member (6) comprises one or more fixing means (16, 17) for the fixing engagement, wherein the fixing means (16, 17) is/are preferably formed as recesses or bumps on the circumferential surface.

8. The conveying device according to the preceding claim, **characterised in that** the second conveying member (5) comprises one or more fixing counter means (14, 15) for the fixing engagement with the fixing means (16, 17) of the blocking member (6).

9. The conveying device according to any one of the preceding four claims, **characterised in that** the blocking member (6) and the second conveying member (5) are axially fixed to each other in the fixing engagement.

10. The conveying device according to any one of the preceding claims, **characterised in that** in the blocking position, the blocking member (6) radially supports the second thread (9) in a positive fit only, with zero force, as long as the threaded engagement is not axially loaded, and preferably supports it radially inwards, or presses it into the threaded engagement, preferably radially outwards.

11. The conveying device according to any one of the preceding claims, **characterised in that** the piston rod (5, 6) can be axially fixed to the piston or is releasably fixed to the piston or is in an axial pressing contact with the piston which preferably only acts in an advancing direction, for conveying.

12. The conveying device according to any one of the preceding claims, **characterised in that** the blocking member (6) forms a front end for contacting the piston.

13. The conveying device according to any one of the preceding claims, **characterised in that** the blocking member (6) protrudes out of the second conveying member (5) and can be axially moved out of an initial position, deeper into the conveying member (5), into the blocking position.

14. The conveying device according to any one of the preceding claims, **characterised in that** the second thread (9) in the flexible axial portion (11, 12) consists of one or more arc segment(s) which extends or which each extend over an angle of at most 180° about the threaded axis (R), wherein if there are a number of arc segments, they are respectively separated from each other on the left and right in the circumferential direction.

15. The conveying device according to any one of the preceding claims, **characterised in that** the second thread (9) is formed on one or more axially extending arm(s) (11, 12) of the flexible axial portion.

16. The conveying device according to the preceding claim, **characterised in that** the second conveying member (5) comprises axially extending breaches (13) between arc segments which are adjacent in the circumferential direction, said breaches (13) separating the arc segments from each other.

17. The conveying device according to any one of the preceding claims, **characterised in that** the second conveying member (5) is hollow at least in the flexible axial portion (11, 12).

18. The conveying device according to any one of the preceding claims, **characterised in that** the second thread (9) is an outer thread.

19. The conveying device according to any one of the preceding claims, **characterised in that** the first thread (8) is an inner thread.

20. The conveying device according to any one of the preceding claims, **characterised in that** the second thread (9) only extends over a part of the axial length of the first thread (8).

21. The conveying device according to any one of the preceding claims, **characterised in that** one of the conveying members (4, 5), preferably the first conveying member (4), can be rotated about the threaded axis (R) relative to the base (1, 2), and the other conveying member can be axially and linearly moved relative to the base (1, 2), but is secured against rotating in relation to the threaded axis (R).

22. The conveying device according to any one of the preceding claims, comprising a drive (3) for automatically rotary-driving one (4) of the conveying members (4, 5).

## Revendications

1. Dispositif pour un appareil servant à l'administration d'un produit, le dispositif comprenant
a) une base (1, 2)
b) un piston pour le transport du produit et une tige de piston pour une poussée du piston,
c) un premier organe de transport (4) mobile par rapport à la base (1, 2) doté d'un premier filetage (8),
d) un deuxième organe de transport (5) mobile par rapport à la base (1, 2) doté d'un deuxième filetage (9) pour une prise filetée avec le premier filetage (8),
e) dans lequel le deuxième filetage (9) s'étend sur une section axiale (11, 12), radialement déformable par rapport à son axe de filetage (R), du deuxième organe de transport (5), si bien qu'un des organes de transport (4, 5) peut être inséré axialement dans l'autre par chevauchement des filetages (8, 9),
f) et un organe de blocage (6) mobile par rapport à la base (1, 2), dans lequel le deuxième organe de transport (5) et l'organe de blocage (6) forment ensemble un télescope et l'un parmi l'organe de blocage (6) et le deuxième organe de transport (5) peut être déplacé axialement de manière télescopique par rapport à l'autre dans une position de blocage dans laquelle l'organe de blocage (6) rigidifie radialement le deuxième filetage (9) dans la prise filetée au niveau de la section axiale déformable (11, 12) et assure ainsi la prise filetée,
**caractérisé en ce que**
g) le deuxième organe de transport (5) et l'organe de blocage (6) forment ensemble la tige de piston (5, 6) mobile le long de l'axe de filetage (R).

2. Dispositif selon la revendication précédente, **caractérisé en ce que** l'organe de blocage (6) ou le deuxième organe de transport (5) peut être déplacé dans le sens axial dans la position de blocage, est de préférence guidé de manière axialement linéaire.

3. Dispositif selon l'une quelconque des revendications précédentes, **caractérisé en ce que** le deuxième organe de transport (5) loge l'organe de blocage (6).

4. Dispositif selon l'une quelconque des revendications précédentes, **caractérisé en ce que** l'organe de blocage (6) est guidé de manière mobile rectiligne axiale bloqué en rotation par rapport à l'axe de filetage (R), de préférence par le deuxième organe de transport (5).

5. Dispositif selon l'une quelconque des revendications précédentes, **caractérisé en ce que** l'organe de blocage (6) est fixé dans la position de blocage axialement par rapport au deuxième organe de transport (5) dans une prise de fixation, de préférence dans une prise d'encliquetage.

6. Dispositif selon l'une quelconque des revendications précédentes, **caractérisé en ce que** l'organe de blocage (6) peut être déplacé d'une prise de fixation désolidarisable, de préférence prise d'encliquetage, par rapport au deuxième organe de transport (5) à la position de blocage.

7. Dispositif selon l'une quelconque des deux revendications précédentes, **caractérisé en ce que** l'organe de blocage (6) présente au niveau d'une surface périphérique un ou plusieurs moyens de fixation (16, 17) pour la prise de fixation, le ou les moyens de fixation (16, 17) étant réalisés de préférence sous forme de creux ou de saillies au niveau de la surface périphérique.

8. Dispositif selon la revendication précédente, **caractérisé en ce que** le deuxième organe de transport (5) présente un ou plusieurs contre-moyens de fixation (14, 15) pour la prise de fixation avec le ou les moyen(s) de fixation (16, 17) de l'organe de blocage (6).

9. Dispositif selon l'une quelconque des quatre revendications précédentes, **caractérisé en ce que** l'organe de blocage (6) et le deuxième organe de transport (5) sont fixés axialement l'un contre l'autre dans la prise de fixation.

10. Dispositif selon l'une quelconque des revendications précédentes, **caractérisé en ce que** l'organe de blocage (6) supporte dans la position de blocage le deuxième filetage (9) uniquement par correspondance de forme radialement sans effort, aussi longtemps que la prise filetée n'est pas sollicitée axialement, et de préférence le supporte radialement vers l'intérieur, ou le pousse dans la prise filetée, de préférence radialement vers l'extérieur.

11. Dispositif selon l'une quelconque des revendications précédentes, **caractérisé en ce que** la tige de piston (5, 6) peut être fixée axialement sur le piston ou y est fixée de manière amovible ou est de préférence en contact de pression axial efficace seulement dans le sens de la poussée pour le transport avec le piston.

12. Dispositif selon l'une quelconque des revendications précédentes, **caractérisé en ce que** l'organe de blocage (6) forme une extrémité avant pour une mise en contact du piston.

13. Dispositif selon l'une quelconque des revendications précédentes, **caractérisé en ce que** l'organe de blocage (6) dépasse du deuxième organe de transport (5) et peut être déplacé d'une position de départ axialement plus profondément dans le deuxième organe de transport (5) jusqu'à la position de blocage.

14. Dispositif selon l'une quelconque des revendications précédentes, **caractérisé en ce que** le deuxième filetage (9) dans la section axiale déformable (11, 12) se compose d'un ou de plusieurs segment(s) en forme d'arc qui s'étend ou s'étendent autour de l'axe de filetage (R) sur un angle de 180° maximum ou respectivement de 180° maximum, dans le cas de plusieurs segments en forme d'arc, ceux-ci sont séparés les uns des autres respectivement à gauche et à droite dans le sens périphérique.

15. Dispositif selon l'une quelconque des revendications précédentes, **caractérisé en ce que** le deuxième filetage (9) est formé au niveau d'un ou de plusieurs bras (11, 12) à extension axiale de la section axiale déformable.

16. Dispositif selon la revendication précédente, **caractérisé en ce que** le deuxième organe de transport (5) présente des passages (13) qui s'étendent axialement entre des segments en forme d'arc adjacents dans le sens périphérique et séparent les segments en forme d'arc les uns des autres.

17. Dispositif selon l'une quelconque des revendications précédentes, **caractérisé en ce que** le deuxième organe de transport (5) est creux du moins dans la section axiale déformable (11, 12).

18. Dispositif selon l'une quelconque des revendications précédentes, **caractérisé en ce que** le deuxième filetage (9) est un filetage extérieur.

19. Dispositif selon l'une quelconque des revendications précédentes, **caractérisé en ce que** le premier filetage (8) est un tauradage.

20. Dispositif selon l'une quelconque des revendications précédentes, **caractérisé en ce que** le deuxième filetage (9) s'étend seulement sur une partie de la longueur axiale du premier filetage (8).

21. Dispositif selon l'une quelconque des revendications précédentes, **caractérisé en ce que** l'un des organes de transport (4, 5), de préférence le premier organe de transport (4), peut tourner par rapport à la base (1, 2) autour de l'axe de filetage (R) et l'autre organe de transport peut être déplacé par rapport à la base (1, 2) de manière axialement linéaire bloqué en rotation par rapport à l'axe de filetage (R).

22. Dispositif selon l'une quelconque des revendications précédentes, comprenant un entraînement (3) pour un entraînement rotatif automatique d'un (4) des organes de transport (4, 5).
